Europäisches Patentamt

European Patent Office

Office européen des brevets

(19)

(11) Veröffentlichungsnummer: **0 006 544**
**B1**

(12) # EUROPÄISCHE PATENTSCHRIFT

(45) Veröffentlichungstag der Patentschrift:
04.11.81

(21) Anmeldenummer: 79101946.6

(22) Anmeldetag: 15.06.79

(51) Int. Cl.³: **A 61 F 1/00**, A 61 B 17/18,
A 61 C 8/00, B 22 F 7/00,
B 32 B 15/04

(54) Implantierbare Knochenersatzwerkstoffe auf der Basis von Calciumphosphat-Keramik in einem Matrix-Material und Verfahren zu deren Herstellung.

(30) Priorität: 23.06.78 DE 2827529

(43) Veröffentlichungstag der Anmeldung:
09.01.80 Patentblatt 80/1

(45) Bekanntmachung des Hinweises auf die Patenterteilung:
04.11.81 Patentblatt 81/44

(84) Benannte Vertragsstaaten:
AT BE CH FR GB IT LU NL SE

(56) Entgegenhaltungen:
DE-A-2 006 010
DE-A-2 127 843
DE-A-2 546 824
DE-A-2 620 907
DE-A-2 621 384
DE-A-2 725 665
DE-A-2 733 394

(73) Patentinhaber: Battelle-Institut e.V., Am Römerhof 35,
D-6000 Frankfurt/Main 90 (DE)

(72) Erfinder: Heide, Helmut, Dr., Am Hohenstein 14,
D-6233 Kelkheim (DE)
Erfinder: Poeschel, Eva, Dr., Sodener Weg 15,
D-6231 Altenhain (DE)
Erfinder: Roth, Ulrich,, Pfannmüllerweg 39,
D-6100 Darmstadt (DE)

(74) Vertreter: Blum, Klaus-Dieter, Dipl.-Ing., Am
Römerhof 35, D-6000 Frankfurt/Main 90 (DE)

MECHANICAL ENGINEERING
Band 100, Nr. 5, Mai 1978,
New York
»Bioactive Materials for Bone, Tooth Implants«,
Seite 60
MEDIZINAL-MARKT/ACTA MEDICOTECHNICA
25. Jahrgang, Nr. 7, 1977,
Berlin,
M. SEMLITSCH et al.
»Bedeutung der Werkstoffe in der biomedizinischen Technik«
Seiten 217 bis 220

Implantierbare Knochenersatzwerkstoffe auf der Basis von Calciumphosphat-Keramik in einem Matrix-Material und Verfahren zu deren Herstellung

Die Erfindung bezieht sich auf implantierbare Knochenersatzwerkstoffe auf der Basis von vollständig oder teilweise resorbierbaren, in einem Matrixmaterial eingelagerten Partikeln aus bioaktiver, gesinterter Calciumphosphat-Keramik der Zusammensetzung $CaO : P_2O_5$ zwischen 3 : 1 und 4 : 1. Verfahren zur Herstellung des Knochenersatzwerkstoffes gehören ebenfalls zur Erfindung.

Implantate aus massiven oder porösen, gewebeverträglichen Metallen oder Metallverbindungen sind bereits bekannt. Z. B. in der DE-A-2 127 843 wird ein Werkstoff beschrieben, der aus einem Metallkern mit einer porösen Beschichtung aus demselben Metall besteht. Die inneren Porenoberflächen können mit bioaktiven Substanzen behandelt werden. Die Poren müssen vorhanden sein, um ein Hineinwachsen des Knochengewebes in die Implantatoberfläche zu ermöglichen. Ferner sind Knochenersatzwerkstoffe bekannt, die aus einer Kunststoffmatrix mit eingelagerten Calciumphosphatpartikeln bestehen (DE-A-2 620 890, 2 620 891, 2 620 907). Verbundwerkstoffe aus Biokeramik und anderen Materialien als Polymerwerkstoffe, insbesondere Metalle, sind dagegen nicht beschrieben. Offensichtlich nahm man bisher an, daß ein anderer Verbundwerkstoff entweder als Knochenersatzwerkstoff ungeeignet ist oder nicht hergestellt werden kann. Bei Verwendung von Metallen wurden daher bioaktive Stoffe immer in Form einer Beschichtung aufgebracht und die Nachteile eines mechanisch weniger belastbaren Übergangs zwischen Metallkern und Beschichtung in Kauf genommen.

Die heute gebräuchlichen Verfahren und Werkstoffe zur Erzeugung eines belastbaren Prothesen-Knochenverbundes beruhen vorwiegend von der Methodik her gesehen auf rein mechanischen Verankerungen, nämlich Verschraubungen und Nagelungen, oder auf rein mechanisch wirksamer Zementierung, z. B. eines Prothesenschaftes, in einer künstlich erzeugten Höhlung des Knochens. Diese mechanisch wirksamen Verankerungsmethoden führen im Knochenlager oft zu unzulässig hohen unphysiologischen Spannungszuständen, die eine Atrophie der betroffenen Knochenregion und somit eine spätere Lockerung der Prothese verursachen (Spannungsatrophie).

Die Stabilität der Prothesenverankerung wird außer von diesen rein mechanischen Phänomenen auch vom Chemismus der verwendeten Materialien beeinflußt. Die heute verwendeten gewebeverträglichen Metalle sowie die biostabilen Oxidkeramiken und die chemisch weitgehend stabilen, gewebeneutralen Polymerwerkstoffe werden vom Knochen ausnahmslos als Fremdkörper erkannt und bleiben als solche gewebig abgekapselt. Diese als eine erste Stufe der Abstoßung zu wertende Erscheinung führt im Falle von dauernd belasteten Prothesenteilen im Knochenimplantatübergangsbereich zur Ausweitung der bindegewebigen Membrane und zur Lockerung und späteren Abstoßung des implantierten Knochenersatzwerkstoffes.

Ein völlig andersartiges physiologisches Verhalten zeigen die bioaktiven Implantationsmaterialien, z. B. die bioaktiven Calciumphosphat-Keramiken, die je nach Zusammensetzung, mehr oder weniger biodegradabel bzw. resorbierbar sind. Sie geben im Zuge ihres chemischen Abbaues an das umliegende Gewebe Stoffe ab, die die Knochenneubildung nicht stören, sondern im Gegenteil eine Calcifizierung des Knochengewebes unmittelbar auf der alloplastischen Oberfläche zulassen oder sogar stimulieren.

Der Begriff »bioaktiv« beinhaltet also per definitionem eine gewisse chemische Reaktion des Werkstoffes mit den Knochenzellen. So wünschenswert diese Eigenschaft für eine direkte Knochenimplantatverbundbildung ist, so schließt sie doch eine alleinige Verwendung dieser Werkstoffe für ein Dauerimplantat aus. Ein weiterer Nachteil dieser bioaktiven Keramik ist ihre relativ geringe mechanische Festigkeit, die ebenfalls einer alleinigen Verwendung für hochbelastbare Endoprothesen entgegensteht. Daher wurden die eingangs erwähnten Knochenersatzwerkstoffe entwickelt, die aus einer nicht resorbierbaren Kunststoffmatrix bestehen, in die Partikel aus Biokeramik eingelagert sind. Für viele Anwendungsfälle läßt jedoch die mechanische Festigkeit dieses Werkstoffes, insbesondere nach Resorption des Keramikanteils, immer noch zu wünschen übrig.

Die Erfindung, wie sie in den Ansprüchen gekennzeichnet ist, löst die Aufgabe, einen implantierbaren Knochenersatzwerkstoff zu schaffen, der die Bioaktivität der in den letzten Jahren bekanntgewordenen Calciumphosphat-Keramiken besitzt und außerdem eine im Vergleich zu den bekannten Implantaten wesentlich höhere mechanische Festigkeit und chemische Stabilität, insbesondere chemische Langzeitstabilität, aufweist.

Gemäß einer Ausführungsform besteht der erfindungsgemäße Knochenersatzwerkstoff aus einem massiven Kern aus gewebeverträglichem Metall, in dessen Randzonen, die als Kontaktflächen mit dem Körpergewebe dienen, Calciumphosphat-Keramik in Form einer einlagigen Schicht aus Partikeln der Größenordnung zwischen ca. 500 µm und einigen Millimetern derart eingelagert sind, daß die Calciumphosphatpartikel an der Oberfläche des Werkstoffes offen liegen. Gemäß einer anderen Ausführungsform ist die Calciumphosphat-Keramik in Form von gleichmäßig verteilten Partikeln der Größenordnung zwischen 20 und 100 µm in einer Matrix aus gewebeverträglichem Metall, und zwar inner-

halb des gesamten Matrixmaterials eingelagert, wobei der Volumenanteil an Calciumphosphat-Keramik in dem Metall zwischen 10 und 30% liegt. Nach einer weiteren Ausführungsart besteht der Werkstoff aus einem massiven Kern aus gewebeverträglichem Metall, der mit einer aufgespritzten Beschichtung aus diesem Metall mit eingelagerter feinpartikulärer Calciumphosphat-Keramik (cermetische Schicht) versehen ist, wobei in dieser Beschichtung der Volumenanteil an Calciumphosphat-Keramik zwischen 10 und 40% liegt. Auf weitere vorteilhafte Ausführungsarten der Erfindung sowie auf Verfahren zur Herstellung solcher Knochenersatzwerkstoffe weisen die beigefügten Unteransprüche 2, 3 und 6 sowie 7 bis 14 hin.

Der erfindungsgemäße Knochenersatzwerkstoff nützt die chemische und mechanische Stabilität der gewebeverträglichen Metalle, z. B. des Titans, und kombiniert diese erwünschten Eigenschaften mit der für die Knochenneubildung und Bildung des Knochenverbundes so wichtigen Bioaktivität von gesinterten Calciumphosphat-Keramiken. Durch Mischung oder Kombination verschiedener Calciumphosphat-Keramiken läßt sich sogar eine abgestufte Resorbierbarkeit erzeugen.

Der erfindungsgemäße Knochenersatzwerkstoff läßt sich durch entsprechende Formgebung für vielfältige Anwendungsgebiete auf dem Gebiet der Implantologie, z. B. als Knochendefektüberbrückungsmaterial, als Befestigungs- und Verschraubungselement zur Verankerung belasteter Gelenkendoprothesen oder implantierbarer Zähne usw. verwenden. Insbesondere zeigt sich der mit dem erfindungsgemäßen Werkstoff gegebene technische Fortschritt in denjenigen Anwendungsfällen, bei denen hohe örtliche Belastungen auftreten.

Weitere Merkmale, Vorteile und Anwendungsmöglichkeiten der Erfindung gehen aus der nachstehenden Beschreibung weiterer Details sowie aus den beigefügten Darstellungen und Schilderungen von Ausführungsbeispielen hervor.

Es zeigen in schematischer Vereinfachung

Fig. 1 eine Ausführungsart des erfindungsgemäßen Knochenersatzwerkstoffes mit grobpartikulärer Calciumphosphat-Keramik,

Fig. 2 die Ausführungsart nach Fig. 1 mit zusätzlichen feinpartikulären Einlagerungen,

Fig. 3 eine Ausführungsart des erfindungsgemäßen Knochenersatzwerkstoffes mit ausschließlich feinpartikulären Einlagerungen,

Fig. 4 eine weitere Ausführungsart, bei der ein metallischer Kern mit einer Schicht aus Metallmatrix, die feinpartikuläres Calciumphosphat und Calciumphosphatkugeln enthält, versehen ist,

Fig. 5 eine Ausführungsart ähnlich Fig. 4, jedoch mit einer aufgespritzten, mehrlagigen Beschichtung mit feinpartikulären Einlagerungen,

Fig. 6 eine Gußform zur Herstellung des Werkstoffes nach Fig. 1 und

Fig. 7 eine viskoelastische Form zur Herstellung des erfindungsgemäßen Knochenersatzwerkstoffes nach Fig. 1, 2, 3 oder 4 durch isostatisches Pressen und anschließendes Sintern oder durch isostatisches Heißpressen.

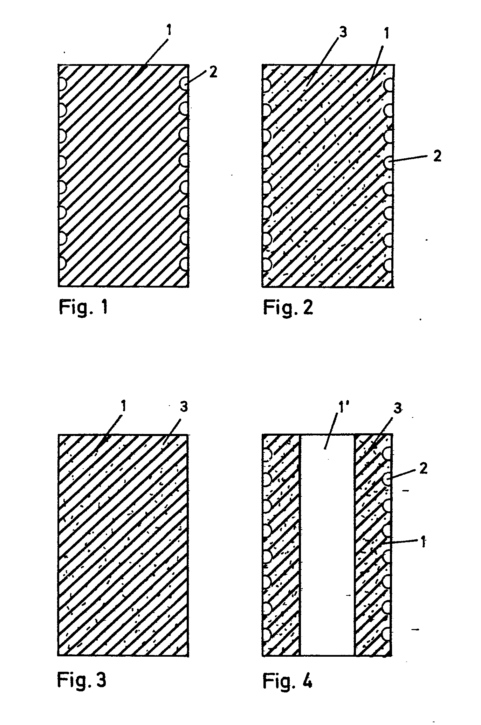

Bei der in Fig. 1 dargestellten Ausführungsart der Erfindung handelt es sich um einen Knochenersatzwerkstoff mit massivem Kern 1 mit grobpartikulären Einlagerungen 2 in den Randzonen. Kernwerkstoff und Matrixmaterial ist ein reines, gewebeverträgliches Metall, z. B. Titan. Die Calciumphosphat-Keramik lag ursprünglich in kugelförmigen Teilchen vor, die beim mechanischen Nachbearbeiten des Formteils, um die Calciumphosphat-Keramik freizulegen und dadurch die Kontaktzonen Calciumphosphat — Knochengewebe zu schaffen, abgeflacht wurden.

Durch das auf der Oberfläche des Ersatzstoffes freiliegende Calciumphosphat wird die direkte, übergangslose Knochenneubildung auf der Implantatoberfläche stimuliert. Unmittelbar nach dieser Primärfixierung des Knochenersatzwerkstoffes bzw. der Prothese beginnt nun die Resorption des bioaktiven Materials und eine simultane Knochenneubildung in den Hohlräumen an der Randzone der Metallmatrix. Hierdurch wird eine endgültige Fixierung des implantierten Prothesenteils eingeleitet.

Gegenüber der bekannten Verwendung poröser Titanimplantate besitzt der erfindungsgemäße Knochenersatzwerkstoff u. a. die entscheidenden Vorteile, daß die Primärfixierung durch die bioaktiven Anteile in sehr kurze Zeit erfolgt (je nach Implantationsort, Alter des Patienten usw. in wenigen Tagen) und daß die bei porösen Werkstoffen der bekannten Art gefürchtete Penetration von Blut in den Implantatkörper, die die Einsprossung von Knochengewebe verhindert, hier nicht eintreten kann.

Bei der Ausführungsart der Erfindung nach Fig. 2 sind zusätzlich zu der grobpartikulären Keramik 2 im Randbereich feinpartikuläre Einlagerungen 3 aus gesinterter Calciumphosphat-Keramik vorhanden. Es handelt sich hier also um einen »Hybridverbundwerkstoff«, der die Vorteile grobpartikulärer und feinpartikulärer Einlagerungen 2 bzw. 3 vereint. Durch die Anwesenheit des feindispersen Calciumphosphat-Anteils wird die gesamte Prothesenoberfläche, einschließlich der Innenwandung der Makroporen, mit einer bioaktiven Grenzfläche ausgestattet, was in vielen Anwendungsfällen zu einer noch schnelleren Prothesenverankerung führen wird. Diese Ausführungsform hat insbesondere den Vorteil, daß die Bildung von calcifiziertem Gewebe (Knochen) auch in tiefer liegenden Implantatbereichen sowie in (nicht gezeigten) kleineren Poren auf der Innenfläche der Makroporen 2 erfolgt. Ein auf diese Weise entstandener Verbund zwischen Knochengewebe und implantiertem Knochenersatzwerkstoff nach Fig. 1 oder 2 ist außerdem imstande, die an der Grenzfläche bei Belastung enstehenden Kräfte so aufzunehmen, daß lokal keine unzulässig hohen Spannungszustände mehr entstehen. Diese Ausführungsart der Erfindung wird also zweckmäßigerweise dort eingesetzt, wo am Implantat-Knochen-Interface hohe Scherbelastungen auftreten können, z. B. beim künstlichen Hüftgelenk.

Bei der Ausführungsart der Erfindung nach Fig. 3 enthält die Metallmatrix sowohl im Inneren des Werkstoffes 1 als auch in den Randzonen in annähernd gleichmäßiger Verteilung feinpartikuläre Einlagerungen 3 aus gesinterter Calciumphosphat-Keramik. Die Teilchengröße sollte etwa zwischen 20 und 100 µm liegen; der Volumenanteil von Calciumphosphat-Keramik im Metall ist je nach Anwendungsfall zwischen etwa 10 und 30% zu variieren.

Der Knochenersatzwerkstoff nach Fig. 4 besteht in seinem Inneren aus reinem, gewebeverträglichem Metall und besitzt eine Randzone, die sowohl feinpartikuläre (3) als auch grobpartikuläre (2) Einlagerungen in einer Metallmatrix 1 enthält. Matrixmetall 1 und Kernmaterial 1' sind in der Regel identisch.

Die Calciumphosphat-Keramik besteht in dem hier beschriebenen Beispiel aus einer Mischung von Tri- und Tetracalciumphosphat, die eine abgestufte Resorbierbarkeit besitzt, weil die Tricalciumphosphatkomponente vollständig, die Tetracalciumphosphatkomponente dagegen kaum resorbierbar ist. Durch diese Maßnahmen behält der Knochenersatzwerkstoff auf Dauer seine bioaktiven Eigenschaften. Diese Werkstoffart stimuliert den Knochen zu einer direkten Verwachsung mit dem Implantat. Obwohl bei der Ausführungsform nach Fig. 3 keine der besseren Verzahnung dienenden größeren Hohlräume vorliegen, dürfte diese Ausführungsart der Erfindung überall dort Einsatz finden, wo aus rein geometrischen Gründen grobpartikuläre Einlagerungen 2 in den Randzonen nicht möglich sind; das gilt insbesondere für Kleinprothesen, z. B. Zahnimplantate, Fingerprothesen usw. Wichtig ist diese Variante der Erfindung auch zur Herstellung von implantierbaren Schrauben und Nägeln, die zur Dauerfixierung dienen. Größere Poren sind bei solchen Werkstücken, z. B. im Bereich der Gewindeflanken, aus Funktionsgründen nicht zulässig.

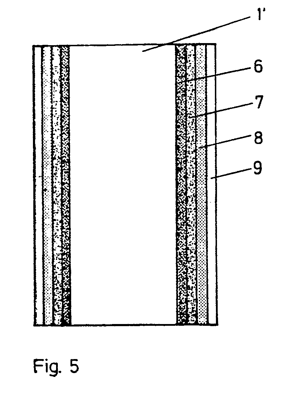

In der Ausführungsart nach Fig. 5 besteht der Kern 1' des Knochenersatzwerkstoffes wiederum aus reinem Metall, auf den eine mehrlagige Beschichtung 6−9 aus dem gleichen Metall und aus eingelagerter, feinpartikulärer Calciumphosphat-Keramik aufgespritzt ist. Die innere Lage 6 ist in diesem Falle ein reinmetallische, poröse Haftschicht, während die übrigen Lagen 7−9 mit unterschiedlichem Volumenanteil, von innen nach außen ansteigend, Calciumphosphat-Partikel enthalten; in der äußersten Schicht 9 beträgt in diesem Beispiel der Calciumphosphat-Anteil nahezu 40%, wodurch eine ausreichende Stabilität und gleichzeitig eine hohe Bioaktivität des Implantats erreicht wird. Es ist auch vorstellbar, daß die äußerste Schicht fast vollständig aus Calciumphosphat-Keramik besteht, wenn dies —

bei einigen Anwendungsgebieten — im Hinblick auf die mechanische Belastbarkeit in Kauf genommen werden kann.

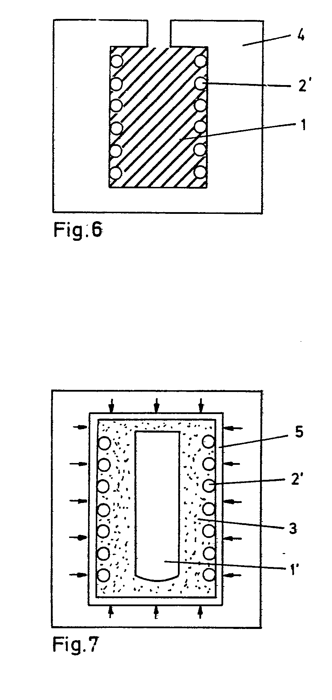

Ein vorteilhaftes Verfahren zur Herstellung des erfindungsgemäßen Werkstoffes läßt sich anhand der Fig. 6 erläutern. Mit 4 ist in Fig. 6 eine Gußform aus einem bekannten feuerfesten Formenmaterial symbolisiert. Auf der Innenwandung dieser Form 4 wird zunächst eine einlagige Schicht aus Tricalciumphosphatkugeln 2' mit einem Durchmesser von 0,5 bis 1 mm mittels eines Klebers fixiert. Sodann wird die Gußform 4 mit schmelzflüssigem Metall 1 gefüllt. Nach dem Erstarren wird das Gußteil mechanisch nachbearbeitet, um die Tricalciumphosphatkugeln 2' an der Oberfläche des gegossenen Werkstückes freizulegen; dadurch werden zwangsläufig die Kugeln 2' zur Implantatoberfläche hin abgeflacht. Ein eventuelles Schmelzen der Calciumphosphatkugeln läßt sich verhindern, wenn die Gußform 4 schnell abgekühlt wird.

Ein anderer Herstellungsweg wird im folgenden anhand Fig. 7 beschrieben. Zur Herstellung eines Knochenersatzwerkstoffes wie nach Fig. 4 wird in diesem Beispiel eine Form 5 aus viskoelastischem Kunststoff verwendet, beispielsweise aus Silikonkautschuk, an deren Innenwandung wiederum als erstes die Calciumphosphatkugeln 2' angeklebt werden. In diese Form 5 wird dann um den Metallkern 1' herum eine Mischung aus Metallpulver und feinpartikulärem Tricalciumphosphatpulver 3, eventuell gemischt mit Tetracalciumphosphatpulver, eingebracht und sodann das Pulver durch isostatisches Pressen verdichtet. Zur Erzielung der notwendigen Stabilität des Implantatwerkstoffes wird der Preßling anschließend in einer Inertgasatmosphäre, beispielsweise in Argon, bei 800 bis 1000°C dicht gesintert. Der so verdichtete Preßling wird dann mechanisch nachbearbeitet, um die grobpartikulären Calciumphosphateinlagerungen 2' an der Oberfläche freizulegen und um das Implantat auf das erforderliche Maß zu bringen. Wird reines Metallpulver nach dem Ankleben der groben Partikel 2' eingefüllt und verpreßt, erhält man den Werkstoff gemäß der Ausführungsart nach Fig. 1.

Als weitere pulvermetallurgische Verfahren können das Heißpressen und das isostatische Heißpressen (HIP) für die Herstellung von Prothesenteilen aus dem erfindungsgemäßen implantierbaren Knochenersatzwerkstoff herangezogen werden.

Beim isostatischen Heißpressen z. B. wird vorteilhafterweise eine Form aus einer dünnen Stahlfolie gebildet, an deren Innenwand wiederum Calciumphosphatkugeln 2' angeklebt werden und zusätzlich eine dünne Schicht Calciumphosphatpulver als Trennmittel aufgebracht wird. In die so vorbereitete Form wird wiederum ein Gemisch aus Metallpulver und feinpartikulärem Calciumphosphat 3 eingefüllt, die Form wird evakuiert und zugeschweißt. Bei erhöhter Temperatur wird die Form einem hohen Gasdruck ausgesetzt, der die Verdichtung des eingefüllten Pulvers zu einem kompakten Bauteil bewirkt. Anschließend wird die Folie abgelöst und durch mechanische Nachbearbeitung werden die Calciumphosphatkugeln in der Oberfläche freigelegt.

Die an sich bekannten Verfahren des Heißpressens und des isostatischen Heißpressens lassen sich zur Herstellung der erfindungsgemäßen Werkstoffe auch in folgender Weise ausführen:

Zunächst wird der Kern aus massivem Metall, z. B. Titan, mit oder ohne feinpartikuläre Einlagerungen — je nach Verwendungszweck — hergestellt, sodann auf diesem Werkstoff Calciumphosphatkugeln in einlagiger Schicht fixiert und schließlich die Zwischenräume zwischen den Kugeln mit pulverförmigem Matrixmetall ausgefüllt. Danach schließt sich dann als nächster Arbeitsgang das Verdichten des Pulvers in der zuvor beschriebenen Weise an.

Nach den beschriebenen Herstellungsmethoden läßt sich der erfindungsgemäße Knochenersatzwerkstoff in vielfältiger Gestalt herstellen. Die makroskopischen, einlagigen Einlagerungen sind jeweils nur an den Flächen vorzusehen, die nach der Implantation mit Körpergewebe in Kontakt gelangen.

Weitere spezielle Herstellungs- und Anwendungsbeispiele werden im folgenden erläutert:

### Beispiel 1

In einem speziellen Fall wurde als Implantat eine Hüftgelenksendoprothese hergestellt. Bei diesem Implantat wird auf den aufgerauhten (Sandstrahlen) Metallkern des Prothesenschaftes zunächst eine metallische Haftschicht aus dem gleichen Basismaterial, aus dem die Prothese besteht, aufgespritzt und dann ein stufenweiser mehrschichtiger cermetischer Aufbau, der 10—40 Vol.-% feinpartikuläre Calciumphosphat-Keramikteilchen enthält, durch Plasmaspritzen aufgebracht (vgl. Fig. 5).

### Beispiel 2

In einem anderen Fall wurden Knochenschrauben zur permanenten Fixierung, mit einer dünnen (ca. 50—200 µm starken) cermetischen Schicht, bestehend aus ca. 10—40 Vol.-% feinpartikulärer Calciumphosphat-Keramik und 90—60 Vol.-% osteokompatiblem Metallpulver mittels Plasmaspritzen beschichtet.

### Beispiel 3

Ein Zahnwurzelimplantat aus gewebeverträglichem Metall, z. B. Titan, wurde durch Plasmaspritzen mit einer ca. 500 µm dicken cermetischen Schicht aus feinpartikulärer Calciumphosphat-Keramik und biokompatiblem Metall im Volumenverhältnis von 30 : 70% versehen.

Das Plasmaspritzen erfolgte mittels einer handelsüblichen Plasmaspritzanlage.

### Patentansprüche

1. Implantierbarer Knochenersatzwerkstoff auf der Basis von vollständig oder teilweise resorbierbaren, in einem Matrixmaterial eingelagerten Partikeln aus bioaktiver, gesinterter Calciumphosphat-Keramik der Zusammensetzung $CaO : P_2O_5$ zwischen 3 : 1 und 4 : 1, dadurch gekennzeichnet, daß der Werkstoff aus einem massiven Kern aus gewebeverträglichem Metall (1, 1') besteht, in dessen Randzonen, die als Kontaktflächen mit dem Körpergewebe dienen, die Calciumphosphat-Keramik in Form einer einlagigen Schicht aus Partikeln (2) der Größenordnung zwischen ca. 500 µm und einigen Millimetern derart eingelagert sind, daß die Calciumphosphatpartikel (2) an der Oberfläche des Werkstoffes offen liegen.

2. Knochenersatzwerkstoff nach Anspruch 1, dadurch gekennzeichnet, daß zusätzlich Calciumphosphat-Keramik in feinpartikulärer Form (3), d. h. in Partikelgrößen zwischen ca. 20 und 100 µm, in den Randzonen eingelagert ist (Fig. 4).

3. Knochenersatzwerkstoff nach Anspruch 1, dadurch gekennzeichnet, daß zusätzlich Calciumphosphat-Keramik (3) in feinpartikulärer Form, d. h. in Partikelgrößen zwischen ca. 20 und 100 µm, innerhalb des gesamten Matrixmetalles (1) in annähernd gleichmäßiger Verteilung eingelagert ist, wobei der Volumenanteil an feinpartikulärer Calciumphosphat-Keramik (3) in dem Metall (1) zwischen 10 und 30% liegt (Fig. 2).

4. Implantierbarer Knochenersatzwerkstoff auf der Basis von vollständig oder teilweise resorbierbaren, in einem Matrixmaterial eingelagerten Partikeln aus bioaktiver, gesinterter Calciumphosphat-Keramik der Zusammensetzung $CaO : P_2O_5$ zwischen 3 : 1 und 4 : 1, dadurch gekennzeichnet, daß die Calciumphosphat-Keramik in Form von gleichmäßig verteilten Partikeln (3) der Größenordnung zwischen 20 und 100 µm in einer Matrix aus gewebeverträglichem Metall (1), und zwar innerhalb des gesamten Matrixmaterials eingelagert ist, wobei der Volumenanteil an Calciumphosphat-Keramik in dem Metall zwischen 10 und 30% liegt (Fig. 3).

5. Implantierbarer Knochenersatzwerkstoff auf der Basis von vollständig oder teilweise resorbierbaren, in einem Matrixmaterial eingelagerten Partikeln aus bioaktiver, gesinterter Calciumphosphat-Keramik der Zusammensetzung $CaO : P_2O_5$ zwischen 3 : 1 und 4 : 1, dadurch gekennzeichnet, daß der Werkstoff aus einem massiven Kern (1') aus gewebeverträglichem Metall besteht, der mit einer aufgespritzten Beschichtung (6—9) aus diesem Metall mit eingelagerter feinpartikulärer Calciumphosphat-Keramik (cermetische Schicht) versehen ist, wobei in dieser Beschichtung (6—9) der Volumenanteil an Calciumphosphat-Keramik zwischen 10 und 40% liegt (Fig. 5).

6. Knochenersatzwerkstoff nach Anspruch 5, dadurch gekennzeichnet, daß die Beschichtung (6–9) mehrlagig aufgebaut ist und daß der Volumenanteil an Calciumphosphat-Keramik in dem Metall der einzelnen Lagen von 0% in der innersten Lage (6) bis zu einem Anteil zwischen 30 und 40% in der äußersten Lage (9) ansteigt.

7. Verfahren zur Herstellung eines Knochenersatzwerkstoffes nach Anspruch 1, dadurch gekennzeichnet, daß an die Innenwandung einer Gußform (4) kugelförmige Calciumphosphatpartikel (2') angeklebt und sodann die Form in dem Matrixmetall (1) ausgegossen wird.

8. Verfahren nach Anspruch 7, dadurch gekennzeichnet, daß durch schnelle Abkühlung der Gußform beim Eingießen das Schmelzen der Calciumphosphatpartikel (2') verhindert wird.

9. Verfahren nach Anspruch 7 oder 8, dadurch gekennzeichnet, daß der Werkstoff zum Freilegen der Calciumphosphatpartikel (2') in den Randzonen, die als Kontaktflächen mit dem Körpergewebe dienen, nach Entnahme aus der Gußform (4) mechanisch nachbearbeitet wird.

10. Verfahren zur Herstellung von Werkstoffen nach Anspruch 1, dadurch gekennzeichnet, daß die Calciumphosphatpartikel (2') durch Ankleben an die Innenwandung einer Form (5) fixiert werden und daß sodann das Matrixmetall (1) als Pulver zugegeben und durch Heißpressen oder isostatisches Heißpressen verdichtet wird.

11. Verfahren nach Anspruch 10, dadurch gekennzeichnet, daß das Matrixmetallpulver mit feinpartikulärer Calciumphosphat-Keramik im Volumenanteil zwischen 10 und 30% vermischt ist.

12. Verfahren zur Herstellung der Knochenersatzwerkstoffe nach Anspruch 1, dadurch gekennzeichnet, daß die Calciumphosphatpartikel (2') auf einen Kern aus dem Matrixmetall aufgeklebt und zusammen mit Matrixmetallpulver, das die Zwischenräume füllt, in eine viskoelastische Form (5) gegeben, dort vorverdichtet und anschließend aufgesintert oder direkt durch isostatisches Heißpressen aufgesintert werden.

13. Verfahren zur Herstellung von Knochenersatzwerkstoffen nach Anspruch 4, dadurch gekennzeichnet, daß eine feindisperse Mischung aus Metallpulver mit feinpartikulärem Calciumphosphat durch Heißpressen oder isostatisches Heißpressen verdichtet wird.

14. Verfahren zur Herstellung von Knochenersatzwerkstoffen nach Anspruch 5 oder 6, dadurch gekennzeichnet, daß die bestehende Beschichtung lageweise durch Flamm- oder Plasmaspritzen aufgebracht wird.

## Claims

1. An implantable bone replacement material based on completely or partially resorbable particles incorporated in a matrix material, the particles being made of bioactive sintered calcium phosphate ceramic material having a CaO : $P_2O_5$ composition ratio between 3 : 1 and 4 : 1, characterized in that the material consists of a solid core of a metal (1, 1') compatible with body tissue containing calcium phosphate ceramic material in the form of a monolayer of particles in its peripheral areas which serve as interfaces to the body tissue, the particles (2) having a size range between about 500 μm and a few millimeters, and the calcium phosphate particles (2) being located so that they are exposed on the outer surface of the material.

2. A bone replacement material as claimed in Claim 1 wherein additional calcium phosphate ceramic material (3) in fine-grained form i. e. with a particle size range between about 20 and 100 μm, is incorporated in the peripheral areas (Figure 4).

3. A bone replacement material as claimed in Claim 1 wherein additional fine-grained calcium phosphate ceramic material (3), with a particle size range between about 20 and 100 μm, is present in approximately uniform distribution throughout the whole matrix metal (1) and wherein the volume of fine-grained calcium phosphate ceramic (3) in the metal (1) is between 10 and 30 percent (Figure 2).

4. An implantable bone replacement material based on completely or partially resorbable particles incorporated in a matrix material, the particles being made of bioactive sintered calcium phosphate ceramic material having a CaO : $P_2O_5$ composition ratio between 3 : 1 and 4 : 1, characterized in that the calcium phosphate ceramic is incorporated in a matrix which is made form metal (1) compatible with body tissue, the ceramic being in the form of uniformly distributed particles (3) in a size range between 20 and 100 μm, and the volume of the calcium phosphate ceramic in the metal being between 10 and 30 percent (Figure 3).

5. An implantable bone replacement material based on completely or partially resorbable particles incorporated in a matrix material, the particles being made of bioactive sintered calcium phosphate ceramic material having a CaO : $P_2O_5$ composition ratio between 3 : 1 and 4 : 1, characterized in that the material consists of a solid core (1') of a metal compatible with body tissue, and having a coating (6–9) of the metal with fine-grained inclusions of calcium phosphate ceramic material, the coating having been applied to the core by spraying, and the coating (6–9) containing between 10 and 40 volume percent of the calcium phosphate ceramic material (Figure 5).

6. Bone replacement material as claimed in Claim 5 wherein the coating (6–9) has a multilayer structure and wherein the volume of the calcium phosphate ceramicmaterial in the metal of the individual layers of the coating rises from zero percent in the first layer (6) to between 30 and 40 percent in the outermost layer (9).

7. A process for the production of the bone replacement material of Claim 1, comprising affixing or pasting spherical calcium phosphate particles (2') to the inner wall of a mold (4), pouring or casting the matrix metal (1) into the mold.

8. Process as claimed in Claim 7 wherein melting of the calcium phosphate particles (2') is prevented by rapid cooling of the mold during casting.

9. Process as claimed in Claim 7 or 8 wherein the material is mechanically worked after its removal from the mold (4) in order to expose the calcium phosphate particles (2') at the periphery, the calcium phosphate particles serving as interfaces to the body tissue.

10. Process for the production of the bone replacement material of Claim 1 comprising affixing or pasting the calcium phosphate particles (2') to the inner wall of a mold (5), adding the matrix metal (1) in powder form and compacting by hot pressing or hot isostatic pressing.

11. Process as claimed in Claim 10 wherein between 10 and 30 volume percent of fine-grained calcium phosphate ceramic material is mixed with the matrix metal powder.

12. Process for the production of the bone replacement material of Claim 1 comprising affixing or pasting the calcium phosphate particles (2') to a core made of matrix metal, and placing it in a viscoelastic mold (5) together with matrix metal powder which fills the empty spaces, then precompacting and finally sintering the materials, or directly sintering by hot isostatic pressing.

13. Process for the production of the bone replacement material according to Claim 4, comprising compacting a finely dispersed mixture of metal powder and fine-grained calcium phosphate by hot pressing or hot isostatic pressing.

14. Process for the production of the bone replacement material according to Claim 5 or 6, comprising applying the coating, which consists of calcium phosphate ceramic material and metal that is compatible with body tissue, one layer after another by flame or plasma spraying.

## Revendications

1. Matériau de prothèse osseuse pour implant, à base de particules totalement ou partiellement résorbables, incorporées dans un matériau de matrice, particules constituées par une céramique bioactive, frittée, de phosphate de calcium, de composition $CaO/P_2O_5$ comprise entre 3/1 et 4/1, matériau de prothèse caractérisé en ce qu'il consiste en un noyau massif en métal compatible avec le tissu du corps humain (1, 1'), dans les zones de bord duquel, qui servent de surfaces de contact avec le tissu du corps, est insérée la céramique de phosphate de calcium, sous la forme d'une couche unique de particules (2) d'un ordre de grandeur de grosseur compris entre environ 500 microns et quelques millimètres, de telle manière que ces particules se trouvent découvertes sur la surface supérieure du matériau.

2. Matériau de prothèse osseuse suivant la revendication 1, caractérisé en ce qu' une céramique de phosphate de calcium, en forme de fines particules (3) de grosseur comprise entre environ 20 et 100 microns, est insérée en supplément dans les zones de bord.

3. Matériau de prothèse suivant la revendication 1, caractérisé en ce que la céramique de phosphate supplémentaire (3) est insérée sous la forme de fines particules, c'est-à-dire de grosseur comprise environ entre 20 et 100 microns, à l'intérieur de l'ensemble du métal de matrice (1) en une répartition sensiblement uniforme, la proportion de la céramique en fines particules (3) dans le métal (1) étant comprise entre 10 et 30%.

4. Matériau de prothèse osseuse à base de particules de céramique de phosphate de calcium, totalement ou partiellement résorbables, bioactives et frittées, d'une composition $CaO/P_2O_5$ comprise entre 3/1 et 4/1, insérées dans un matériau de matrice, caractérisé en ce que la céramique de phosphate de calcium, en forme de particules uniformément réparties, d'une grosseur de l'ordre de 20 à 100 microns, est insérée dans une matrice en métal (1) compatible avec le tissu du corps humain, et à l'intérieur de la totalité du matériau de matrice, la proportion en volume de la céramique de phosphate de calcium dans le métal étant comprise entre 10 et 30%.

5. Matériau de prothèse osseuse à base de particules de céramique de phsophate de calcium, totalement ou partiellement résorbables, bioactives et frittées, d'une composition $CaO/P_2O_5$ comprise entre 3/1 et 4/1 insérées dans un matériau de matrice, caractérisé en ce que le matériau consiste en un noyau massif (1') de métal compatible avec le tissu humain, qui est pourvu d'un revêtement pulvérisé au pistolet (6–9) en ce même métal, avec des particules fines de céramique de calcium insérées dans le métal (couche cermétique), la proportion en volume de céramique de phosphate de calcium dans ce revêtement étant comprise entre 10 et 40%.

6. Matériau de prothèse suivant la revendication 5, caractérisé en ce que le revêtement (6–9) est constitué en plusieurs couches, et la proportion en volume de céramique de phosphate de calcium dans le métal des dufférentes couches croît depuis 0% dans la couche la plus intérieure (6) jusqu'à une proportion comprise entre 30 et 40% dans la couche la plus extérieure (9).

7. Procédé pour la fabrication d'un matériau pour prothèse osseuse suivant la revendication 1, caractérisé en ce que, sur la paroi intérieure d'un moule de coulée (4), on colle des particules de phosphate de calcium de forme sphérique, et ensuite on remplit le moule avec le métal de matrice.

8. Procédé suivant la revendication 7, caractérisé en ce que, par une refroidissement rapide du moule lors de la coulée, on s'oppose à une fusion des particules de céramique de phosphate de calcium (2').

9. Procédé suivant l'une des revendications 7 et 8, caractérisé en ce que le matériau, après sa sortie du moule (4) est travaillé mécaniquement pour libérer les particules de céramique de phosphate de calcium dans les zones de bord qui servent de surfaces de contact avec le tissu du corps humain.

10. Procédé suivant la revendication 1, caractérisé en ce que les particules de phosphate de calcium (2') sont fixées par collage à la paroi intérieure du moule (5) et ensuite le métal de matrice (1) est ajouté comme une poudre et est compacté par pressage à chaud ou pressage à chaud isostatique.

11. Procédé suivant la revendication 10, caractérisé en ce que la poudre de matrice est mélangée avec la céramiq   de phosphate de calcium en fines particules,    s une proportion en volume comprise entre 10 et 30%.

12. Procédé suivant la revendication 1, caractérisé en ve que les particules de phosphate de calcium (2') sont collées sur un noyau en métal de matrice, et sont introduites, en commun avec une poudre de métal de matrice, qui remplit les espaces intermédiaires, dans un moule (5) à viscosité élastique, où le mélange est compacté et finalement fritté, ou directement fritté par pressage à chaud isostatique.

13. Procédé suivant la revendication 4, caractérisé en ce qu'un mélange finement dispersé de poudre de métal et de phosphate de calcium en fines particules est compacté par pressage à chaud ou pressage à chaud isostatique.

14. Procédé suivant l'une des revendications 5 ou 6, caractérisé en ce que le revêtement en céramique de phosphate de calcium et de métal compatible avec tissus humains est déposé par couche par pulvérisation à la flamme ou au plasma.

Fig. 1

Fig. 2

Fig. 3

Fig. 4

Fig. 5

Fig.6

Fig.7